(19) Europäisches Patentamt
European Patent Office
Office européen des brevets

(11) **EP 3 733 105 B1**

(12) **EUROPEAN PATENT SPECIFICATION**

(45) Date of publication and mention
of the grant of the patent:
**23.10.2024  Bulletin 2024/43**

(51) International Patent Classification (IPC):
***A61B 34/10*** *(2016.01)*    ***A61B 6/02*** *(2006.01)*
***A61B 6/00*** *(2024.01)*    ***A61B 6/50*** *(2024.01)*

(21) Application number: **19171754.5**

(22) Date of filing: **30.04.2019**

(52) Cooperative Patent Classification (CPC):
**A61B 34/10; A61B 6/02; A61B 6/481; A61B 6/486;**
**A61B 6/488; A61B 6/504; A61B 6/5217;**
A61B 6/4441; A61B 6/503; A61B 2034/104;
A61B 2034/105

(54) **METHOD AND SYSTEM FOR DETERMINING PRESSURE TO ACHIEVE INFLATION OF AN OCCLUSION DEVICE**

VERFAHREN UND SYSTEM ZUM BESTIMMEN DES DRUCKS ZUM ERREICHEN DES AUFBLASENS EINER OKKLUSIONSVORRICHTUNG

PROCÉDÉ ET SYSTÈME POUR DÉTERMINER LA PRESSION DE GONFLAGE D'UN DISPOSITIF D'OCCLUSION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**04.11.2020  Bulletin 2020/45**

(73) Proprietor: **Siemens Healthineers AG**
**91301 Forchheim (DE)**

(72) Inventors:
• **Munoyat, Rahul Bharath**
**560040 Bangalore, Karnataka (IN)**

• **Chirag, Sharma**
**560100 Bangalore (IN)**

(74) Representative: **Bals & Vogel Patentanwälte**
**PartGmbB**
**Sendlinger Strasse 42A**
**80331 München (DE)**

(56) References cited:
**WO-A1-2016/132164    US-A1- 2013 261 601**

**Description**

The present invention relates to a method and system for determining a pressure value to achieve inflation of an occlusion device.

Incidences of cardiac imaging procedures are on the rise in modern times. This may be due to vascular damage caused by vascular ailments such as stenosis or restenosis. Medical procedures such as balloon angioplasty may have to be performed on patients to widen the lumen of the occluded blood vessels. Such procedure may be performed using an occlusion device such as a balloon catheter. It is important in such medical procedures that accurate determination of lumen diameter and possible increase in the lumen diameter be made.

Currently, there is no quantifiable way to determine the percentage opening of lumen diameter achievable for an occluded blood vessel, i.e. determination of the final lumen diameter post balloon angioplasty is not determinable. Additionally, the pressure to be applied to the occlusion device is dependent on the expertise of a physician performing the medical procedure. If the occlusion device is inflated more than a defined threshold, damage may be caused to the occluded blood vessel. Additionally, under-inflation of the occlusion device may not allow efficient expansion of the blood vessel. Therefore, determination of pressure to be applied to the occlusion device may be error-prone and time consuming. Related prior art is disclosed in document WO 2016/132164 A1.

The object of the invention is therefore to accurately determine the inflation pressure for the occlusion device so as to enable efficient expansion of the lumen of the occluded blood vessel.

The invention achieves the object by a method and a system for determining a pressure to achieve inflation of an occlusion device.

The present invention describes a method of determining a pressure to achieve inflation of an occlusion device. The method includes a processing unit-implemented step of obtaining at least one medical image of an occlusion in an occluded blood vessel. The occlusion may be, for example, a blockage in the blood vessel which narrows a lumen diameter of the occluded blood vessel. At least one medical image of the occlusion may be obtained, for example, using a medical imaging device. In an embodiment, multiple medical images of the occlusion may be obtained at various angles such that the occlusion is imaged accurately and characteristics associated with the occlusion are identifiable. In another embodiment, such medical image may be obtained without contrast or dye. The method further includes a step of determining by an image processor one or more characteristics associated with the occlusion, from the at least one medical image. The one or more characteristics may be, for example, position/location, size, thickness, and composition of the occlusion. The characteristics associated with the occlusion enable determination of properties of the occlusion. Therefore, accurate determination of the pressure for inflation of the occlusion device is enabled. The method further includes determining pressure to be applied to inflate the occlusion device based on the one or more characteristics associated with the occlusion. For example, thickness of the occlusion may enable determination of how much pressure may be applied to inflate the occlusion device to achieve extension of the diameter of the occluded blood vessel. The occlusion may be composed of one or more materials of varying elasticity. Determination of such properties of the occlusion further enables accurate determination of inflation pressure of the occlusion device. In an embodiment, inflation of the occlusion device causes an increase in the diameter of the blood vessel. Advantageously, accurate determination of the inflation pressure of the occlusion device enables efficient expansion of the occluded blood vessel. Therefore, application of additional pressure on the occluded blood vessel may be prevented thereby preventing damage to the blood vessel.

According to an embodiment of the invention, determining one or more characteristics associated with the occlusion includes processing unit-implemented steps of identifying from the least one medical image one or more sub-regions of the occlusion. In an embodiment, the occlusion may have one or more sub-regions depending on a composition of the occlusion. The occlusion may be composed of, for example, one or more materials distinct from one another. The occlusion may also be a combination of one or more distinct materials. The materials may be, for example, but not limited to, lipid plaque, fibrous plaque and calcified plaque. Such layers of distinct material may form a sub-region in the occlusion. Each of the sub-region may have a different elastic property, i.e. application of pressure to the sub-region may cause expansion or reduction in thickness of the sub-region. The method further includes identifying one or more material properties of the one or more sub-regions of the occlusion. In an embodiment, the occlusion may include only one material. Advantageously, identification of one or more sub-regions of the occlusion enables efficient determination of inflation pressure of the occlusion device. The method further includes determining a thickness of each of the sub-regions of the occlusion. Determination of thickness of each of the sub-region enables identification of pressure required to be applied to cause a reduction in the thickness of the sub-region so as to increase the lumen diameter of the occluded blood vessel. In an embodiment, a thickness of the occlusion may be determined if there are no sub-regions in the occlusion.

According to an embodiment of the invention, in determining one or more characteristics associated with the occlusion, the method includes identifying one or more material properties of the occlusion. The occlusion may be composed of, for example, a single material of a defined elasticity. Identification of the material of the occlusion enables identification of elasticity of the occlusion. The method further includes determining a thickness of the occlusion. Determination of thickness of the occlusion enables identification of pressure required to be applied to cause a compression of the occlusion. This enables an increase the lumen diameter of the occluded blood vessel.

According to the invention, determining the pressure to be applied to inflate the occlusion device includes processing unit-implemented steps of determining an actual diameter of the occluded blood vessel. The actual diameter of the occluded blood vessel may include the diameter of the blood vessel without the occlusion. The diameter of the blood vessel may be determined by calculating a distance between two points on two sides of the blood vessel, wherein a line between such two points passes through a center point of the blood vessel. Determination of the actual diameter of the occluded blood vessel may enable identification of a maximum limit of expansion of the blood vessel using the occlusion device. The method further includes determining a lumen diameter of the occluded blood vessel. Such lumen diameter is less than the actual diameter of the occluded blood vessel. The lumen diameter may be calculated by considering the shortest distance between a point on a surface of the occlusion and a point on a wall opposite to the occlusion in the occluded blood vessel. The method further includes determining a radial pressure applied by the occlusion device. The radial pressure is determined based on at least one material property of the occlusion in the blood vessel. In an embodiment, the radial pressure may be modified in strength depending on the sub-region of the occlusion. For example, the fibrous plaque region of the occlusion may require higher pressure to be compressed in comparison to the lipid plaque sub-region of the occlusion. The method further includes a processing unit-implemented step of simulating a change in the lumen diameter of the occluded blood vessel based on the determined radial pressure. Simulation methods may be a substitute to physical experimentation of a solution. Use of simulation methods may enable prediction of one or more outcomes of an action without actually performing such action in real-time. Simulation methods therefore may enable identification of the most optimal solution for a given scenario. In an embodiment, simulating the change in the lumen diameter of the occluded blood vessel may be based on a mathematical model. The mathematical model may contain one or more parameters of the occluded blood vessel thereby creating a virtual model of the occluded blood vessel. Change in the lumen diameter may be simulated by applying one or more conditions to the mathematical model. In the embodiment, the one or more conditions may be one or more radial pressures to be applied to the occluded blood vessel. The simulation may calculate the outcome of application of each of the one or more radial pressures on the occluded blood vessel, such as the extent to which compression of the occlusion is achieved. The simulation may also enable, for example, determination of any damage to the blood vessel due to excessive compression of the occlusion or over inflation of the occluded blood vessel. Advantageously, simulating the change in the lumen diameter of the occluded blood vessel enables accurate identification of the pressure value for inflation of the occlusion device, before the medical procedure. Therefore, any damage to the blood vessel is avoided.

According to an embodiment which is not part of the claimed invention, determining the pressure to be applied to the occlusion device includes obtaining at least one characteristic associated with the occlusion device. The at least one characteristics may include, but not be limited to, compliance of the occlusion device, simulated pressure versus volume of the occlusion device, failure volume of the occlusion device and deflation rate of the occlusion device. The method further includes determining by the inflation pressure processor a pressure to be applied to the occlusion device based on the at least one characteristic associated with the occlusion device. Advantageously, the pressure to be applied to the occlusion device may be accurately identified not just based on the occlusion properties but also the characteristics of the occlusion device. Therefore, rate of success of the process is increased.

According to yet another embodiment, the method further includes repeating the step of simulating a changed lumen diameter based on an increased radial pressure. Such radial pressure may be increased by a pre-configured value for every repetition, for example, by 10132.5 Pa (0.1 atm) at every iteration. Such pre-configured value of pressure may be based on the material properties of the occlusion. In the simulation process, the initial radial pressure may be chosen to be the minimum pressure required to initiate compression of one of the sub regions of the occlusion. Such sub-region may be preferably the sub region closest to the center of the lumen of the occluded blood vessel. Such radial pressure may be increased by a fixed amount so as to determine the optimal amount of pressure required to achieve maximum compression of such sub-region. The method may include determining a change in the lumen diameter of the occluded blood vessel at every increase in the radial pressure. Increase in the radial pressure may cause a change in the thickness of at least one of the one or more sub-regions of the occlusion. In an embodiment, the radial pressure may be increased for a specific sub-region until the thickness of such sub-region remains constant. The radial pressure may then be increased so as to induce compression of the subsequent sub-region of the occlusion. Advantageously, steady increase in the radial pressure enables accurate determination of the pressure value required for efficient inflation of the occlusion device. Therefore, any damage to the occluded blood vessel in real-time is prevented. Additionally, the invention enables the physician to determine if a target lumen diameter is achieved after application of a defined radial pressure. The method further includes determining a final radial pressure to be applied in the occluded blood vessel. Such final radial pressure achieves an increase in the lumen diameter of the occluded blood vessel to a target lumen diameter. In an embodiment, the final radial pressure may achieve efficient compression of the occlusion in the occluded blood vessel. The final radial pressure may be characterized by the simulation result being a changed lumen diameter that is equal to or greater than a target lumen diameter. The method further includes calculating the pressure to be applied to inflate the occlusion device based on the final radial pressure to be applied to the occluded blood vessel by the occlusion device. For a given cylindrical occluded blood vessel, radial stress provides pressure exerted by an inflated occlusion device on the occlusion. Therefore, the pressure to be applied to inflate the occlusion device may be determined, for example, using a circumferential stress equation for a thin walled cylinder that may be well-known in the art. Advantageously, determination of radial pressure enables accurate determination of pressure value for inflation of the occlusion device. This enables efficient identification of the extent to which the occlusion device may be inflated in the occluded blood vessel, before the medical procedure. The method further includes overlaying the simulated changed lumen diameter of the blood vessel on the occlusion in the at least one medical image. Advantageously, the overlaying the simulated change enables the physician to accurately identify the change in the lumen diameter achieved due to a certain pressure value.

According to an embodiment, the at least one medical image is an X-ray image. Such X-ray image may be obtained without contrast.

According to yet another embodiment, the at least one medical image of the occlusion is obtained at an intensity such that the occlusion region is radio-transparent. The one or more materials in the occlusion may turn radio-transparent at specific X-ray intensities. Therefore, categorization of the occlusion into sub-regions is enabled. Such categorization may be performed using, for example, Hounsfield unit values. X-ray intensity may be converted to Hounsfield unit values using techniques well-known in the art. In an embodiment, the at least one medical image may be chosen such that the X-ray intensity of the medical image has reached a threshold or the occlusion appears radio-transparent.

The object of the invention is also achieved by a system for determining a pressure value to achieve inflation of an occlusion device.

The invention relates in one aspect to a computer program product comprising a computer program, the computer program being loadable into a storage unit of a system, including program code sections to make the system execute the method according to an aspect of the invention when the computer program is executed in the system.

The invention relates in one aspect to a computer-readable medium, on which program code sections of a computer program are saved, the program code sections being loadable into and/or executable in a system to make the system execute the method according to an aspect of the invention when the program code sections are executed in the system.

The realization of the invention by a computer program product and/or a computer-readable medium has the advantage that already existing management systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The present invention is further described hereinafter with reference to illustrated embodiments shown in the accompanying drawings, in which:

| | |
|---|---|
| FIG 1 | illustrates a block diagram of a data processing system in which an embodiment for determining a pressure value to achieve inflation of an occlusion device can be implemented. |
| FIG 2 | illustrates a flowchart of a method of determining a pressure value to achieve inflation of an occlusion device, according to an embodiment. |
| FIG 3 | illustrates a flowchart of a method of determining one or more characteristics associated with the occlusion, according to an embodiment. |
| FIG 4 | illustrates a flowchart of a method of obtaining a medical image, according to an embodiment. |
| FIG 5 | illustrates a flowchart of a method of determining the pressure to be applied to inflate the occlusion device, according to an embodiment. |
| FIG 6 | illustrates cross-sectional images of the occluded blood vessel with the change in radio-transparency of the occlusion with increasing X-ray intensity, according to an embodiment. |
| FIG 7A&B | illustrate graphical representations of change in diameter of the occluded blood vessel before compression of the occlusion and after compression of the occlusion at varying pressure values, according to an embodiment. |
| FIG 8 | illustrates a graphical representation of increase in diameter of the occluded blood vessel with increasing radial pressure applied to the occluded blood vessel, according to an embodiment. |

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

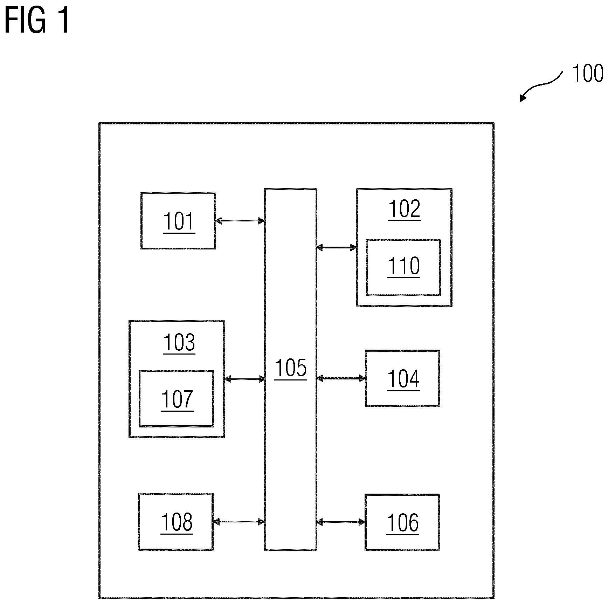

FIG 1 is a block diagram of a data processing system 100 in which an embodiment can be implemented, for example, as a system 100 determining a pressure value to achieve inflation of an occlusion device, configured to perform the processes as described therein. In FIG 1, said data processing system 100 comprises a processing unit 101, a memory 102, a storage unit 103, an input unit 104, an output unit 106, a bus 105, and a network interface 108.

The processing unit 101, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

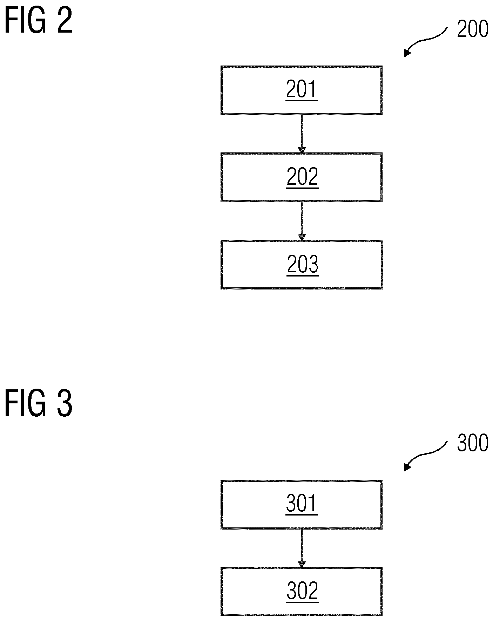

The memory 102 may be volatile memory and non-volatile memory. The memory 102 may be coupled for communication with said processing unit 101. The processing unit 101 may execute instructions and/or code stored in the memory 102. A variety of computer-readable storage media may be stored in and accessed from said memory 102. The memory 102 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 includes a pressure determination 110 stored in the form of machine-readable instructions on any of said above-mentioned storage media and may be in communication to and executed by processor 101. When executed by the processor 101, the pressure determination module 110 causes the processor 101 to determine a pressure value to achieve inflation of an occlusion device. Method steps executed by the processor 101 to achieve the abovementioned functionality are elaborated upon in detail in FIG 2, 3, 4, and 5.

The storage unit 103 may be a non-transitory storage medium which stores a medical database 107. The medical database 107 is a repository of medical images and associated medical data sets related to one or more patients that is maintained by a healthcare service provider. The input unit 104 may include input means such as keypad, touch-sensitive display, camera (such as a camera receiving gesture-based inputs), etc. capable of receiving input signal such as a medical image. The bus 105 acts as interconnect between the processor 101, the memory 102, the storage unit 103, the input unit 204, the output unit 106 and the network interface 108.

Those of ordinary skilled in the art will appreciate that said hardware depicted in FIG 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 100 in accordance with an embodiment of the present disclosure includes an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. Said operating system is modified or created in accordance with the present disclosure as described.

Disclosed embodiments provide systems and methods for processing medical images. In particular, the systems and methods may determine a pressure value to achieve inflation of an occlusion device.

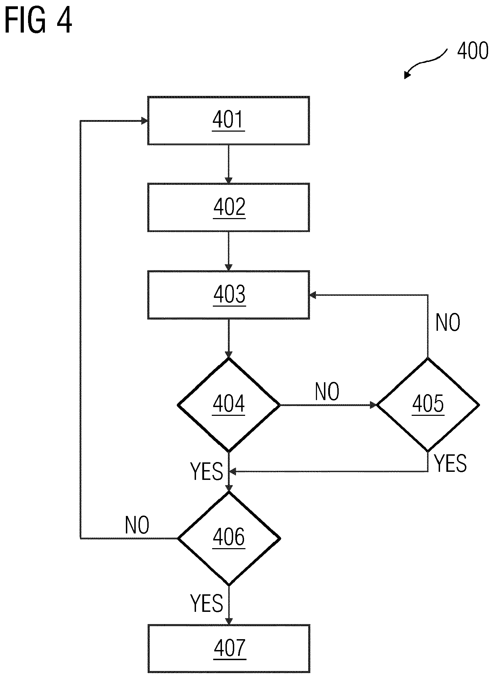

FIG 2 illustrates a flowchart of a method 200 of determining a pressure value to achieve inflation of an occlusion device. At step 201, the method 200 includes obtaining at least one medical image of an occlusion in an occluded blood vessel. An occlusion may be a blockage in a blood vessel which restricts blood flow through the blood vessel. Such blockage may cause narrowing of the blood vessel and may prove to be fatal for a patient. The medical image may be, for example, an X-ray image. In an embodiment, the medical image may be any radiological image depicting the occluded blood vessel. The method steps of obtaining at least one medical image of the occluded vessel are described in detail in FIG 4. Referring to FIG 4, at step 401, C-arm of an X-ray device is positioned at a desired position and angle on a patient such that an X-ray image of the occluded blood vessel may be acquired. At step 402, an X-ray image of the occluded blood vessel is acquired using the X-ray device. In an embodiment, such X-ray image may be obtained without a contrast or dye. At step 403, intensity of X-ray is increased so as to ensure that the occlusion is radiotransparent. A radiotransparent occlusion enables efficient identification of one or more sub-regions of the occlusion. At step 404, a determination is made if the whole region of the occlusion is radiotransparent in the acquired X-ray image. If the whole region of the occlusion is not radiotransparent, a determination is made at step 405 if a threshold of X-ray intensity is reached. If the threshold is not reached at step 405, the intensity of the X-ray is increased further at step 403. If the whole region of the occlusion is radiotransparent and if the threshold of the X-ray intensity is achieved at step 405, at step 406 a determination is made if X-ray images of the occluded blood vessel are acquired at all desired angles. If X-ray images at all desired angles are obtained, the X-ray images are analyzed at step 407 to determine at least one X-ray image which accurately depicts the occlusion in the occluded blood vessel.

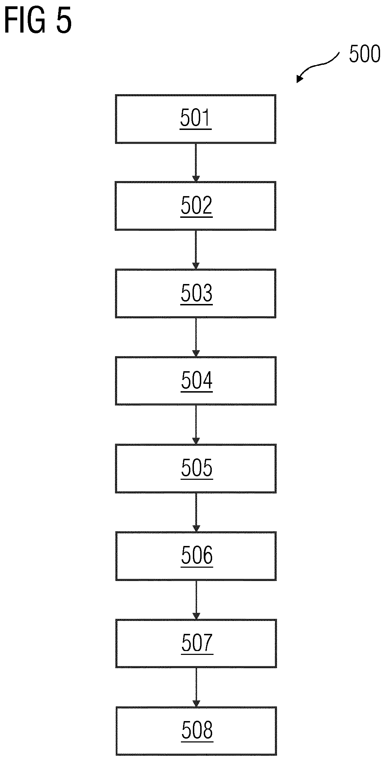

At step 202, one or more characteristics associated with the occlusion are determined. Such characteristics may be, for example, composition, size, thickness, and location of the occlusion. Characteristics of the occlusion enable efficient identification of the pressure to be applied to the occlusion for compression. The method steps to identify one or more characteristics of the occlusion are described in detail in FIG 3. At step 203, pressure to be applied to inflate the occlusion device is determined based on the one or more characteristics associated with the occlusion. The inflation of the occlusion device causes an increase in a diameter of the occluded blood vessel. In an embodiment, the occlusion device may be, for example, a balloon catheter. A balloon catheter is inflated inside a blood vessel such that a compression of one or more occlusion is achieved in the blood vessel. The balloon catheter may also enable clearing of any obstruction in the blood vessel. The method steps to achieve determination of the pressure to be applied to inflate the occlusion device are described in detail in FIG 5.

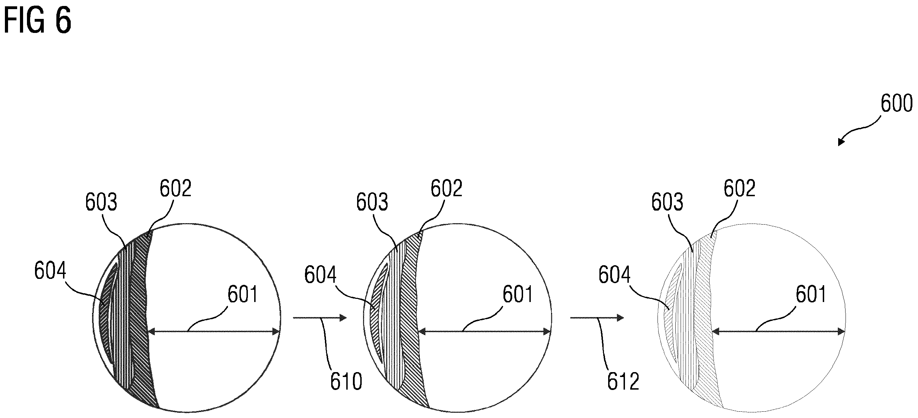

FIG 3 illustrates a flowchart of an embodiment of a method 300 of determining one or more characteristics associated with the occlusion. At step 301, one or more sub-regions of the occlusion are identified from the at least one medical image. Such one or more sub-regions may be composed of a distinct material or a combination of one or more distinct materials. Specific materials turn radiotransparent at specific X-ray intensities thereby enabling categorization of the occlusion into one or more sub-regions. Such categorization may be performed based on Hounsfield unit values. The Hounsfield unit values may be derived from the X-ray intensity of the at least one medical image. FIG 6 illustrates an embodiment of cross-sectional images 600 of the occluded blood vessel with the change in radio-transparency of the occlusion with increasing X-ray intensity. Referring to FIG 6, the cross-sectional images 600 depict an occlusion with three sub-regions, i.e. fibrotic plaque 602, lipidic plaque 603 and calcified plaque 604. The artery lumen 601 is depicted as a distance between the occlusion and vessel wall on the opposite end of the occlusion. When X-ray intensity is increased at step 610, the lipidic plaque 603 turns radiotransparent the quickest. The fibrotic plaque 602 may turn radiotransparent when the X-ray intensity is increased further at step 612. Calcified plaque 604 may turn radiotransparent at higher X-ray intensities. In an embodiment, the occlusion in the blood vessel may be composed of a single material. Therefore, such occlusion may not include more than one regions.

At step 302 of the method 300, a thickness of the occlusion is determined from the radiotransparent image of the occlusion. In an embodiment, each of the sub-regions of the occlusion may be determined from the radiotransparent image of the occlusion. Determination of the thickness of the occlusion or each of the sub-regions of the occlusion enables efficient determination of the pressure to be applied to the occlusion so as to achieve a compression of the occlusion. The thickness of each of the sub-regions of the occlusion may be determined may be, for example, based on the pixel values in each of the sub-regions of the occlusion in the at least one medical image.

FIG 5 illustrates a flowchart of a method 500 of determining the pressure to be applied to inflate the occlusion device, according to an embodiment. At step 501, an actual diameter of the occluded blood vessel is determined. The actual diameter of the occluded blood vessel may be the diameter without the occlusion. Such diameter may be determined, for example, as the longest distance between the opposite walls of the blood vessel passing through the center of the blood vessel. At step 502, a lumen diameter of the occluded blood vessel is determined. Such diameter is less than the diameter of the actual blood vessel. The diameter of the occluded blood vessel may be determined, for example, by considering a shortest distance between a point on a surface of the occlusion and a point on a wall opposite to the occlusion in the blood vessel. In an embodiment, the lumen diameter of the blood vessel may be used to determine a cross-sectional area of the blood vessel. At step 503, a radial pressure to be applied to the occlusion is determined. Such radial pressure is determined based on at least one material property of the occlusion. The pressure to be applied to the occlusion may vary based on the elastic properties of the occlusion. Therefore, greater the elasticity of the occlusion, higher the pressure that may be applied. The elasticity of the occlusion or at least one of the regions of the occlusion may determine the extent to which the occlusion may be compressed. Therefore, with increasing elasticity, greater compression of the occlusion may be achieved. In an embodiment, if the occlusion may include one or more sub-regions, the radial pressure to be applied to the occlusion may be a summation of the pressures applied to achieve compression of each sub-region of the occlusion.

In an embodiment, the radial pressure to be applied to the occlusion may be optimally identified based on the compression achieved in the occlusion. Each sub-region of the occlusion may have a threshold beyond which the sub-region may not compress. Therefore, further increase in the pressure may cause damage to the blood vessel. Thus, the radial pressure is determined such that efficient compression of the occlusion is achieved without causing damage to the blood vessel. At step 504 of the method 500, a change in the lumen diameter of the blood vessel is simulated based on the determined radial pressure. Simulating the change in the lumen diameter of the occluded blood vessel may be based on a mathematical model. The mathematical model may contain one or more parameters of the occluded blood vessel thereby creating a virtual model of the occluded blood vessel. Change in the lumen diameter may be simulated by applying one or more conditions to the mathematical model. In the embodiment, the one or more conditions may be one or more radial pressures to be applied to the occluded blood vessel. The simulation may calculate the outcome of application of each of the one or more radial pressures on the occluded blood vessel, such as the extent to which compression of the occlusion is achieved. The simulation may also enable, for example, determination of any damage to the blood vessel due to excessive compression of the occlusion or over inflation of the occluded blood vessel. Advantageously, simulating the change in the lumen diameter of the occluded blood vessel enables accurate identification of the pressure value for inflation of the occlusion device, before the medical procedure. Therefore, any damage to the blood vessel is avoided.

In an embodiment, the change in the lumen diameter of the blood vessel is determined using the below equation:

$$\mathrm{E} \;=\; \frac{\sigma(\varepsilon)}{\varepsilon} \;=\; \frac{F/A}{\Delta L/\mathrm{L0}} \;=\; \frac{F\mathrm{L0}}{A\Delta L}$$

Where:

E = Young's modulus of elasticity
F = Force exerted on an object under tension
A = Actual cross-sectional area of the blood vessel, which equals area of cross-section perpendicular to the applied force
$\Delta L$ = Amount by which thickness of the occlusion changes
$L_0$ = Original thickness of the occlusion

The simulation of application of the radial pressure to the occlusion may be repeated wherein the radial pressure is increased by a pre-configured amount, for example, 10132.5 Pa (0.1 atm), for every repetition. Such repetition may be performed until an efficient compression of the occlusion is achieved. At step 505, a final radial pressure to be applied to the occlusion is determined. The final radial pressure may be a summation of the pressures applied to the one or more sub-regions of the occlusion. The final radial pressure may a pressure that increases the lumen diameter of the blood vessel to a target lumen diameter. In an embodiment, the target lumen diameter may be a diameter of the blood vessel that enables efficient flow of blood. Such target lumen diameter may be proportional to the diameter of the vessel area surrounding the occluded portion of the blood vessel. At step 506, the pressure to be applied to the occlusion device is calculated based on the final radial pressure to be applied to the occlusion. In an embodiment, the pressure to be applied to the occlusion device may be determined, for example, using a radial stress equation for a thin walled cylinder. Such equation may be well-known in the art and has not been described for the purposes of brevity. The method 500 further includes a step 507 of overlaying the simulated changed lumen diameter of the blood vessel on the occlusion in the at least one medical image. Overlaying the simulated blood vessel enables efficient identification of the most accurate lumen diameter of the blood vessel, and thereby the pressure corresponding to such accurate lumen diameter.

Below is non-limiting embodiment of the determining the pressure to be applied to the occlusion device:
An occlusion in a left anterior descending (LAD) artery is assumed where the original diameter of the blood vessel is 3.5 millimeter. The occlusion may have three sub-regions, viz., hypercellular fibrous plaque, lipidic plaque and cellular fibrotic plaque, wherein the hypercellular fibrous plaque is closest to the vessel lumen and the cellular fibrotic plaque is closest to the vessel wall. The thickness of the hypercellular fibrous plaque ($L_{hcf}$) is 0.9 millimeter, the thickness of the lipidic plaque is 1.2 millimeter and the thickness of the cellular fibrotic plaque is 0.8 millimeter.

Therefore the effective lumen diameter of the occluded blood vessel is 2.9 millimeter. The Young's modulus (E) for hypercellular fibrous plaque, lipidic plaque and cellular fibrotic plaque are 106.8 kPa (106800 $N/m^2$), 9.0 kPa (9000 $N/m^2$), and 16.1 kPa (16100 $N/m^2$). The below equation is used to simulate pressure to be applied to the occlusion from 10132.5 Pa (0.1 atm) (10132.5 $N/m^2$) at increments of 10132.5 Pa (0.1 atm).

$$\mathrm{E} \;=\; \frac{\sigma(\varepsilon)}{\varepsilon} \;=\; \frac{F/A}{\Delta L/\mathrm{L0}} \;=\; \frac{F\mathrm{L0}}{A\Delta L}$$

Based on the change in the thickness of the sub-regions of the occlusion, increase in the lumen diameter is determined.

For first iteration of simulation of increase in pressure, the change in thickness of the plaque(in mm) is calculated for hypercellular fibrous plaque as below:

7

$$\Delta L_{hcf} = pL_{hcf}/Y = (10132.5*0.0009)/106800 = 0.0853862 \text{ mm}$$

Similarly, for each iteration, the pressure to be applied to the occlusion is increased by 10132.5 Pa (0.1 atm) and the change in the lumen diameter is calculated. After a certain pressure value, the change in thickness of the hypercellular fibrous plaque remains constant. Therefore, further increase in pressure may only push the hypercellular fibrous plaque. Thereafter, pressure to be applied to the lipidic plaque and the cellular fibrotic plaque may be simulated.

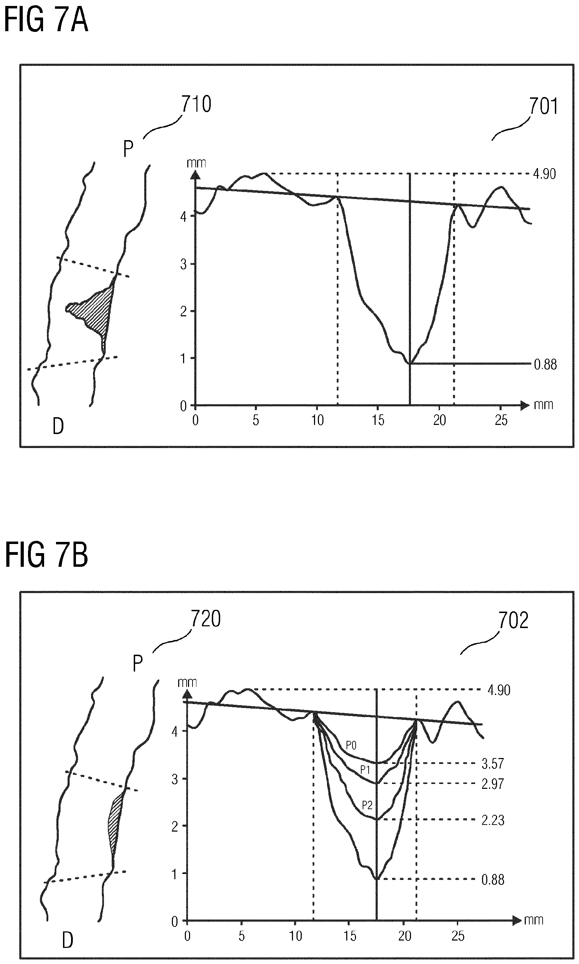

FIG 7A and 7B illustrate graphical representations 701, 702 of change in diameter of the occluded blood vessel before compression of the occlusion 710 and after compression of the occlusion 720 at varying pressure values, according to an embodiment. The graph 701 illustrates a change in the lumen diameter of an occluded blood vessel. The blood vessel 710 indicates presence of an occlusion due to which the diameter of the blood vessel decreases. The point at which the occlusion is the thickest has the lowest lumen diameter. Graph 702 illustrates an increase in the lumen diameter of the occluded blood vessel 720 with increasing pressures applied to the occlusion. The graph 702 indicates the effect of pressures $P_0$, $P_1$, and $P_2$ on the diameter of the blood vessel. With increasing pressures, a compression of the occlusion is achieved, thereby enabling the lumen diameter of the occluded blood vessel to be increased to the target lumen diameter.

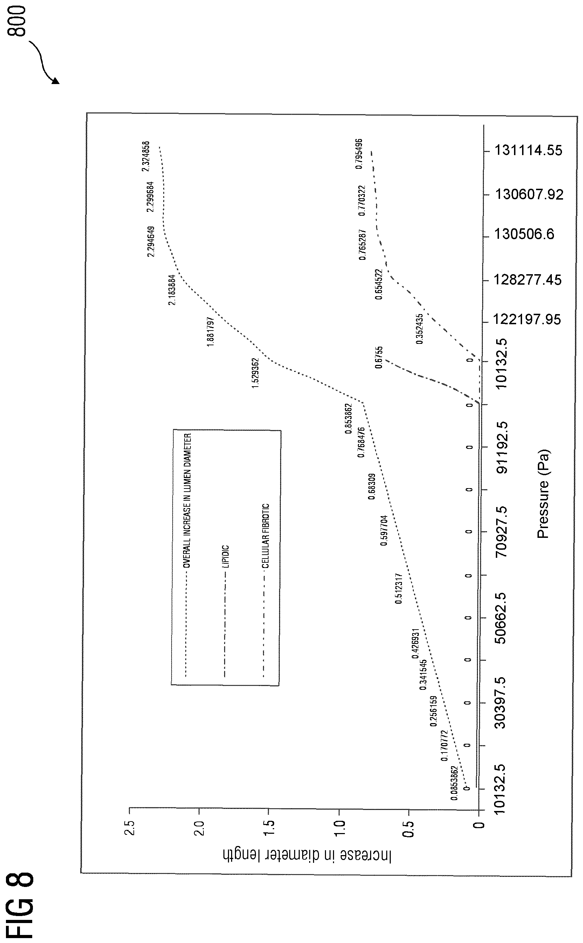

FIG 8 illustrates a graphical representation 800 of increase in diameter of the occluded blood vessel with increasing radial pressure applied to the occluded blood vessel, according to an embodiment. The radial pressure may be the pressure applied ot the occlusion by the occlusion device. The graph 800 depicts the change in the thickness of each of the sub-regions of the occlusion in the blood vessel. Such change in the thickness of the sub-regions enables an overall increase in the lumen diameter of the blood vessel. The thickness of one or more sub-regions of the occlusion may not change unless the thickness of the sub-region closest to the lumen has not reached a threshold.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein.

**Claims**

1. A method (200) of determining a pressure to achieve inflation of an occlusion device, the method (200) comprising processing unit-implemented steps of:

   obtaining (201) at least one medical image of an occlusion in a blood vessel;
   determining (202) by an image processor one or more characteristics associated with the occlusion from the at least one medical image; and
   determining (203) by an inflation pressure processor, the inflation pressure processor being identical to or different from the image processor, a pressure to be applied to the occlusion device based on the one or more characteristics associated with the occlusion,
   **characterised in that**
   determining (203) the pressure to be applied to the occlusion device comprises:
   determining (501) an actual diameter of the occluded blood vessel;
   determining (502) a lumen diameter of the occluded blood vessel; wherein the lumen diameter is less than the actual diameter of the occluded blood vessel;
   determining (503) a radial pressure applied by the occlusion device; wherein the radial pressure is determined based on at least one material property of the occlusion; and
   simulating (504) a changed lumen diameter of the blood vessel based on the determined radial pressure.

2. The method (200) according to claim 1, wherein determining (202) one or more characteristics associated with the occlusion comprises:

   identifying (301) from the at least one medical image one or more sub-regions of the occlusion;
   identifying one or more material properties of the one or more sub-regions of the occlusion; and
   determining (302) a thickness of each of the sub-regions.

3. The method (200) according to claim 1, wherein determining (202) one or more characteristics associated with the occlusion comprises:

identifying the at least one material property of the occlusion; and
determining (302) a thickness of the occlusion.

4. The method (200) according to claim 1, further comprising:

repeating the step of simulating (504) a changed lumen diameter based on an increased radial pressure, wherein the radial pressure is increased by a pre-configured value for every repetition, wherein the pre-configured value of pressure is based on the material properties of the occlusion;
determining (505) a final radial pressure to be applied by the occlusion device; wherein the final radial pressure is **characterized by** the simulation result being a changed lumen diameter that is equal to or greater than a target lumen diameter;
calculating (506) the pressure to be applied to the occlusion device based on the final radial pressure to be applied by the occlusion device; and
overlaying (507) the simulated changed lumen diameter of the blood vessel on the occlusion in the at least one medical image.

5. The method (200) according to any of the abovementioned claims, wherein the at least one medical image is an X-ray image.

6. The method (200) according to any of the abovementioned claims, wherein the at least one medical image is obtained at an intensity so as to make an occlusion region radio-transparent.

7. A system (100) for determining (203) a pressure value to achieve inflation of an occlusion device, the system (100) comprising:

one or more processing units (101);
a medical database coupled to the one or more processing units (101);
a memory (102) coupled to the one or more processing units (101), the memory (102) comprising a pressure determination module configured to perform the method steps as claimed in any one of the claims 1 to 6.

8. A computer program product comprising machine readable instructions, that when executed by a processing unit (101), cause the processing unit (101) to perform a method according to any one of the claims 1-6.

9. A computer readable medium on which program code sections of a computer program are saved, the program code sections being loadable into and/or executable in a system (100) to make the system (100) execute the method of any one of the claims 1 to 6 when the program code sections are executed in the system (100).

**Patentansprüche**

1. Verfahren (200) zur Bestimmung eines Drucks, um ein Aufblasen einer Okklusionsvorrichtung zu erreichen, wobei das Verfahren (200) die folgenden von einer Verarbeitungseinheit implementierten Schritte umfasst:

Erhalten (201) mindestens eines medizinischen Bildes einer Okklusion in einem Blutgefäß;
Bestimmen (202) eines oder mehrerer mit der Okklusion verbundener Merkmale aus dem mindestens einen medizinischen Bild durch einen Bildprozessor; und
Bestimmen (203) eines auf die Okklusionsvorrichtung anzuwendenden Drucks durch einen Aufblasdruckprozessor, wobei der Aufblasdruckprozessor mit dem Bildprozessor identisch oder von diesem verschieden ist, auf der Grundlage der einen oder mehreren mit der Okklusion verbundenen Eigenschaften,

**dadurch gekennzeichnet, dass**
das Bestimmen (203) des auf die Okklusionsvorrichtung anzuwendenden Drucks umfasst:

Bestimmen (501) eines tatsächlichen Durchmessers des verschlossenen Blutgefäßes;
Bestimmen (502) eines Lumendurchmessers des verschlossenen Blutgefäßes, wobei der Lumendurchmesser kleiner ist als der tatsächliche Durchmesser des verschlossenen Blutgefäßes;

Bestimmen (503) eines radialen Drucks, der von der Okklusionsvorrichtung ausgeübt wird, wobei der radiale Druck auf der Grundlage von mindestens einer Materialeigenschaft der Okklusion bestimmt wird; und Simulieren (504) eines veränderten Lumendurchmessers des Blutgefäßes auf der Grundlage des ermittelten radialen Drucks.

2. Verfahren (200) nach Anspruch 1, wobei das Bestimmen (202) eines oder mehrerer mit der Okklusion verbundener Merkmale umfasst:

Identifizieren (301) eines oder mehrerer Unterbereiche der Okklusion aus dem mindestens einen medizinischen Bild;
Identifizieren einer oder mehrerer Materialeigenschaften des einen oder der mehreren Unterbereiche der Okklusion; und
Bestimmen (302) der Dicke jeder der Unterregionen.

3. Verfahren (200) nach Anspruch 1, wobei das Bestimmen (202) eines oder mehrerer mit der Okklusion verbundener Merkmale umfasst:

Identifizieren der mindestens einen Materialeigenschaft der Okklusion; und
Bestimmen (302) einer Dicke der Okklusion.

4. Verfahren (200) nach Anspruch 1, ferner umfassend:

Wiederholen des Schritts des Simulierens (504) eines geänderten Lumendurchmessers auf der Grundlage eines erhöhten radialen Drucks, wobei der radiale Druck bei jeder Wiederholung um einen vorkonfigurierten Wert erhöht wird, wobei der vorkonfigurierte Druckwert auf den Materialeigenschaften der Okklusion basiert;
Bestimmen (505) eines endgültigen radialen Drucks, der von der Okklusionsvorrichtung auszuüben ist; wobei der endgültige radiale Druck **dadurch gekennzeichnet ist, dass** das Simulationsergebnis ein veränderter Lumendurchmesser ist, der gleich oder größer als ein Ziellumendurchmesser ist;
Berechnen (506) des auf die Okklusionsvorrichtung auszuübenden Drucks auf der Grundlage des endgültigen radialen Drucks, der von der Okklusionsvorrichtung ausgeübt werden soll; und
Überlagern (507) des simulierten veränderten Lumendurchmessers des Blutgefäßes mit der Okklusion in dem mindestens einen medizinischen Bild.

5. Verfahren (200) nach einem der vorgenannten Ansprüche, wobei das mindestens eine medizinische Bild ein Röntgenbild ist.

6. Verfahren (200) nach einem der vorstehenden Ansprüche, wobei das mindestens eine medizinische Bild mit einer solchen Intensität gewonnen wird, dass ein Verdeckungsbereich strahlendurchlässig wird.

7. System (100) zum Bestimmen (203) eines Druckwerts, um ein Aufblasen einer Okklusionsvorrichtung zu erreichen, wobei das System (100) umfasst:

eine oder mehrere Verarbeitungseinheiten (101);
eine medizinische Datenbank, die mit der einen oder mehreren Verarbeitungseinheiten (101) verbunden ist;
einen Speicher (102), der mit der einen oder den mehreren Verarbeitungseinheiten (101) verbunden ist, wobei der Speicher (102) ein Druckbestimmungsmodul umfasst, das so konfiguriert ist, dass es die in einem der Ansprüche 1 bis 6 beanspruchten Verfahrensschritte durchführt.

8. Computerprogrammprodukt mit maschinenlesbaren Anweisungen, die, wenn sie von einer Verarbeitungseinheit (101) ausgeführt werden, die Verarbeitungseinheit (101) veranlassen, ein Verfahren nach einem der Ansprüche 1 bis 6 durchzuführen.

9. Computerlesbares Medium, auf dem Programmcodeabschnitte eines Computerprogramms gespeichert sind, wobei die Programmcodeabschnitte in ein System (100) ladbar und/oder ausführbar sind, um das System (100) zu veranlassen, das Verfahren nach einem der Ansprüche 1 bis 6 auszuführen, wenn die Programmcodeabschnitte in dem System (100) ausgeführt werden.

**Revendications**

1. Méthode (200) de déterminant une pression pour obtenir le gonflage d'un dispositif d'occlusion, la méthode (200) comprenant des étapes mises en œuvre par l'unité de traitement comprenant :

   obtenir (201) au moins une image médicale d'une occlusion dans un vaisseau sanguin ;
   déterminer (202) par un processeur d'images une ou plusieurs caractéristiques associées à l'occlusion à partir d'au moins une image médicale ; et
   déterminer (203) par un processeur de pression de gonflage, le processeur de pression de gonflage étant identique ou différent du processeur d'images, une pression à appliquer au dispositif d'occlusion sur la base d'une ou plusieurs caractéristiques associées à l'occlusion,

   **caractérisé en ce que**
   la détermination (203) de la pression à appliquer au dispositif d'occlusion comprend :

   déterminer (501) le diamètre réel du vaisseau sanguin occlus ;
   déterminer (502) le diamètre de la lumière du vaisseau sanguin occlus, le diamètre de la lumière étant inférieur au diamètre réel du vaisseau sanguin occlus ;
   déterminer (503) une pression radiale appliquée par le dispositif d'occlusion, la pression radiale étant déterminée sur la base d'au moins une propriété matérielle de l'occlusion ; et
   simuler (504) une modification du diamètre de la lumière du vaisseau sanguin sur la base de la pression radiale déterminée.

2. Méthode (200) selon la revendication 1, dans laquelle la détermination (202) d'une ou plusieurs caractéristiques associées à l'occlusion comprend :

   identifier (301) à partir d'au moins une image médicale une ou plusieurs sous-régions de l'occlusion ;
   identifier une ou plusieurs propriétés matérielles d'une ou plusieurs sous-régions de l'occlusion ; et
   déterminer (302) l'épaisseur de chacune des sous-régions.

3. Méthode (200) selon la revendication 1, dans laquelle la détermination (202) d'une ou plusieurs caractéristiques associées à l'occlusion comprend :

   identifier au moins une propriété matérielle de l'occlusion ; et
   déterminer (302) l'épaisseur de l'occlusion.

4. Méthode (200) selon la revendication 1, comprenant en outre :

   répéter l'étape consistant à simuler (504) une modification du diamètre de la lumière sur la base d'une augmentation de la pression radiale, la pression radiale étant augmentée d'une valeur préconfigurée à chaque répétition, la valeur préconfigurée de la pression étant basée sur les propriétés matérielles de l'occlusion ;
   déterminer (505) une pression radiale finale à appliquer par le dispositif d'occlusion, la pression radiale finale étant **caractérisée par le fait que** le résultat de la simulation est un diamètre de lumière modifié qui est égal ou supérieur à un diamètre de lumière cible ;
   calculer (506) la pression à appliquer au dispositif d'occlusion en fonction de la pression radiale finale à appliquer par le dispositif d'occlusion ; et
   superposer (507) le diamètre modifié simulé de la lumière du vaisseau sanguin sur l'occlusion dans au moins une image médicale.

5. Méthode (200) selon l'une des revendications susmentionnées, dans laquelle l'au moins une image médicale est une image radiographique.

6. Méthode (200) selon l'une quelconque des revendications précédentes, dans lequel l'au moins une image médicale est obtenue à une intensité telle qu'elle rend radiotransparente une région d'occlusion.

7. Système (100) permettant de déterminer (203) une valeur de pression pour obtenir le gonflage d'un dispositif d'occlusion, le système (100) comprenant :

une ou plusieurs unités de traitement (101) ;
une base de données médicales couplée à une ou plusieurs unités de traitement (101) ;
une mémoire (102) couplée à une ou plusieurs unités de traitement (101), la mémoire (102) comprenant un module de détermination de la pression configuré pour exécuter les étapes du procédé selon l'une quelconque des revendications 1 à 6.

8. Produit de programme informatique comprenant des instructions lisibles par une machine qui, lorsqu'elles sont exécutées par une unité de traitement (101), amènent l'unité de traitement (101) à exécuter une méthode selon l'une des revendications 1 à 6.

9. Support lisible par ordinateur sur lequel sont sauvegardées des sections de code d'un programme d'ordinateur, les sections de code pouvant être chargées et/ou exécutées dans un système (100) pour que le système (100) exécute la méthode de l'une des revendications 1 à 6 lorsque les sections de code sont exécutées dans le système (100) .

FIG 1

FIG 2

200

| 201 |
|:---:|

↓

| 202 |
|:---:|

↓

| 203 |
|:---:|

FIG 3

300

| 301 |
|:---:|

↓

| 302 |
|:---:|

FIG 4

400

# FIG 5

500

```
┌──────────────┐
│     501      │
└──────────────┘
        │
        ▼
┌──────────────┐
│     502      │
└──────────────┘
        │
        ▼
┌──────────────┐
│     503      │
└──────────────┘
        │
        ▼
┌──────────────┐
│     504      │
└──────────────┘
        │
        ▼
┌──────────────┐
│     505      │
└──────────────┘
        │
        ▼
┌──────────────┐
│     506      │
└──────────────┘
        │
        ▼
┌──────────────┐
│     507      │
└──────────────┘
        │
        ▼
┌──────────────┐
│     508      │
└──────────────┘
```

FIG 6

EP 3 733 105 B1

## FIG 7A

## FIG 7B

FIG 8

800

EP 3 733 105 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016132164 A1